Europäisches Patentamt

European Patent Office

Office européen des brevets

(19)

(11) Publication number: **0 092 882**

A1

(12)

# EUROPEAN PATENT APPLICATION

(21) Application number: 83200580.5

(22) Date of filing: 19.04.83

(51) Int. Cl.³: **C 05 F 9/04**
**C 12 P 5/02**

(30) Priority: 21.04.82 NL 8201655

(43) Date of publication of application:
02.11.83 Bulletin 83/44

(84) Designated Contracting States:
AT BE CH DE FR GB IT LI LU NL SE

(71) Applicant: RUTTE RECYCLING B.V.
Osdorperweg 578
NL-1067 SZ Amsterdam(NL)

(72) Inventor: Rutte, Johannes Bartholomeus
Sloterweg 842
NL-1066 CP Amsterdam(NL)

(74) Representative: van der Beek, George Frans et al,
Nederlandsch Octrooibureau Johan de Wittlaan 15 P.O.
Box 29720
NL-2502 LS Den Haag(NL)

(54) A one-step process for the controlled anaerobic fermentation of the organic fraction of municipal solid waste.

(57) The present invention relates to a process wherein in a depot of the organic fraction of municipal solid waste, that has been obtained after removal of non-fermentable components present therein, the humidity is adjusted at at least 40 weight %, the pH-value is adjusted at 6 to 8, and then firstly in a fermentation under aerobic conditions the oxygen present is consumed, secondly carbohydrates, fats and proteins are hydrolyzed under anaerobic conditions, thirdly said hydrolysis products formed are fermented under anaerobic conditions, into methane and carbon dioxide, and the residue formed in the depot is removed.

EP 0 092 882 A1

A one-step process for the controlled anaerobic fermentation of the organic fraction of municipal solid waste.

The present invention relates to a process for the anaerobic fermentation of the organic fraction of municipal solid waste, by subjecting said fraction to a treatment, wherein after a short fermentation under aerobic conditions the oxygen present is consumed at the metabolism of aerobic microorganisms, carbohydrates, fats and proteins are hydrolyzed under anaerobic conditions by means of facultatively anaerobic microorganisms and subsequently the hydrolysis products formed, are fermented, likewise under anaerobic conditions, by means of strictly anaerobic microorganisms, predominantly into methane and carbon dioxide.

From the SVA publication 48 as well as from Gas (11), 563–568 (1981), such a process is known, wherein within a period of several years a sanitary landfill is formed, wherein beyond the microbiologically fermentable organic fraction also a non-neglectable amount of non-microbiologically fermentable materials is present. The anaerobic fermentation that does not run optimally, due to the presence of non-microbiologically fermentable material, runs substantially uncontrolled. Thus the production of biogas is not optimal and it takes a long time to ferment all anaerobically fermentable components of the landfill. Another objection is that after the fermentation of the organic fraction under anaerobic conditions there remains a residue that has a relatively high humidity and contains various materials, that may not be fermented under anaerobic conditions. Sieving of the residue fraction is difficult, since the high humidity may cause a clustering of finely divided material. In combination with the very variable composition of existing landfills, it is for that reason why municipal solid waste processing plants donot process further the formed residue that contains still a lot of compost.

Further Dutch Patent Application 80.01997 and 80.06567 disclose a process wherein first the components of municipal solid waste, that may not be fermented microbiologically, are removed, then in a first reaction step the carbohydrates, fats and proteins that are present in the microbiologically fermentable fraction are hydrolyzed; at said hydrolysis for example water soluble fatty acids are formed that subsequently are elutriated together with other water soluble organic

2    0092882

and inorganic materials by means of an aqueous liquid and the solution formed is fed to at least one auxiliary reactor, wherein in a second reaction step the organic material is fermented under anaerobic conditions know per-se into a blend of carbon dioxide and methane, and the compost formed is removed from the reactor.

In case said process is carried out the household garbage has been degraded sufficiently far within some weeks to be used as a compost. From experimental results it appears that about 75% of the yield of biogas is achieved from the auxiliary reactor(s). Thus the auxiliary reactor is the most important link of the process. If the organic fraction from a sorting plant is used a reduction of organic material of about 55% is achieved. However, said organic material contains just a small amount of wood and paper (corresponding to about 7% of cellulose). The percentage of cellulose in the garbage that is processed according to said process, may not be high since as appears from the experiments described in the Refcom Report (the Refcom Device, that is used in the process of USP 4.022.665, is described by Walter Perron in "Symposium Papers" Energy from biogas and waste IV, 1980, Florida, U.S.A.) cellulose can not be degraded within a period of a couple of weeks.

In British Patent Specification 1,341,305 a process for transforming household garbage into compost is disclosed; said garbage is subjected successively to 3 stages i.c. an aerobic alcoholic fermentation, wherein mineral acid is added to inhibit the activity of acid-forming microorganisms present, an anaerobic fermentation at a humidity of 40-80 % by weight and a pH of 7 to 8, and finally a fermentation during which hydrogen is evolved and methanic fermentation is prevented. Consequently, the purpose and steps 1 and 3 as well differ principally from the process of the present invention.

In USP 2,337,686 a process for the conversion of town refuse into humus fertilizer material is disclosed. In said process in a closed cell said refuse is subjected first to aerobic conditions and then to anaerobic conditions in order to promote multiplication of microorganisms present and consequently to obtain considerable amounts of enzymes to degrade high molecular compounds into lower molecular compounds. When the multiplication has reached the level desired, wherein the organic compounds present are fermented at a temperature

3

0092882

of to 60-65°C and vapours evolved are withdrawn. Consequently, the purpose and step 3 differ principally from the process of the present invention.

It was found now, that an optimally running anaerobic microbiological fermentation of the organic fraction of municipal solid waste, wherein also cellulose is degraded, may be achieved, in case in a depot of the organic fraction, that is obtained after removal of non-fermentable components from municipal solid waste, the humidity is adjusted at at least 40 weight %, the pH is adjusted within the range from 6 up to 8, said conditions are maintained and the degraded residue formed in the depot is removed.

The non-microbiologically fermentable components that are removed from the municipal solid refuse in order to obtain the microbiologically fermentable fraction, consists predominantly of iron, non-ferro metals, glass and coarse refuse.

The microbiological fermentation of the organic fraction according to the process of the invention will be completed within a period of from 7 to 10 years.

Due to the long residence time not only the simply degradable organic waste materials are degraded, but also the components that may be degraded only slowly, such as cellulose, are degraded within said period.

Due to the low requirements that are raised for the refuse that is fed to the depot – organic industrial waste, organic agricultural waste, as well as lime containing waste may be fed directly to the depot – a considerable saving with respect to the preselection may be achieved. In case the household garbage is collected separately e.g. by separating wet and dry sacks, the contents of the wet sack may be fed directly to the depot.

The process is not proper to process hazardous wastes, since said material might inhibit the anaerobic biological activity .

The process is extremely proper for processing limited amounts of dehydrated and wet waste. The advantages have to be seen especially in a propagated start of the biological activity and the possibility for pH-control.

The humidity as well as the pH-value in the depot may be controlled by means of measuring and controlling devices known per-se.

4

0092882

An important advantage is, that the residue contains a very high percentage of organic material and that said organic material may be separated simply from the non-desired components.

At dumping refuse, air is included. This occurs also in case densifying techniques are used.

The oxygen present in the depot is used at the metabolism of the aerobic microorganisms present.

Since just a limited amount of oxygen is present, the aerobic phase is short, i.c. a couple of days up to a couple of weeks.

So the heat produced is insufficient to obtain high temperatures. Consequently, in the anaerobic phase, the average temperature of the refuse in the depot is within the range of from 30 to 40°C.

Since carbon dioxide is formed as a reaction product, the carbon dioxide concentration in the void spaces of the depot may increase up to 90 weight %. Then the carbon dioxide will be dissolved in the water present, so that the carbon dioxide concentration in the void spaces as well as the acidity of the water will decrease.

As the oxygen is consumed further, the phase wherein the facultatively anaerobic microorganisms will become active, will increase.

Said microorganisms elaborate enzymes, that hydrolyse carbohydrates, fats and proteins into soluble low molecular materials, such as glucose and fatty acids having a small number of carbon atoms, e.g. acetic acid, propionic acid and butyric acid.

In said phase also some gaseous reaction products, as e.g. $NH_3$, $H_2$ and $CO_2$ are released. Also due to the high generation rate of the microorganisms, i.c. a couple of hours, the rate of fermentation is high.

Said microorganisms are very tolerant with respect to their environment. Thus variations in temperature and acidity are tolerated well.

Due to the fact that water soluble fatty acids are formed the acidity of the water is still decreased. In said phase pH-values of from 4.5 to 5.0 are usual. As the phase wherein the facultatively anaerobic microorganisms are active has made progress, strictly anaerobic microorganisms become more active. The generation time of said microorganisms is longer than the generation time of the facultatively anaerobic microorgansms, i.c. a couple of days. Contrary to the strictly anaerobic microorganisms said microorganisms are sensitive for variations in temperature, acidity and for the presence of heavy metals. Thus the water present should have a temperature of from 25 to 60°C and a pH from 6 to 8 and the weight percentage of ions of heavy metals in the water should be low. In many landfills said requirements are not met. It is for that reason that the production of biogas is inhibited.

Further the medium should not be merely very reducing in its activity, but also a sufficient amount of materials having a buffering capacity should be dissolved in the water, so that variations in the acidity may be avoided. The optimum pH in said phase should vary from 6.4 to 7.2. Outside of said range the activity will decrease fastly. In said phase formic acid, acetic acid, carbon dioxide and hydrogen are reacted into methane, carbon dioxide and biomass.

Since the generation time of the facultatively anaerobic microorganisms and of the strictly anaerobic microorganisms differs considerably, it takes a lot of time before both processes have been tuned up mutually.

In the beginning of said phase, dependent on the conditions, is achieved after 0.5 up to 2 years of anaerobic fermentation. If the conditions required are maintained said phase may last decades of years.

Since the mesophilic optimum is at 35°C and the thermophilic optimum is at 45°C, it is efficient to maintain the temperature as near as possible to said optimae.

In said phase of the process the water level in the depot should be adjusted as soon as possible at the optimum value. In practice that is 1/3 of the depths of the depot.

The average humidity of municipal solid waste is about 35 weight %. Since it has appeared, that the development of the temperature in the depot is directly dependent on the humidity, the humidity should be preferably at least 55 weight %..

The fermentation of organic material increases considerably as the particle size is reduced. Consequently, it is important, that the organic material is attainable well (actually that means not wrapped by a plastic sack) and is tipped in particles that are as small as possible.

Due to an uncareful tipping technique, dependent on the particle size and the densification, the wetting material might flow away along predetermined ways. This is very disadvantageous for the required uniform wetting of the refuse depot.

The depot is formed preferably in cells wherein the refuse feed of a certain period may be stored. Preferably, cells are used wherein the refuse feed of one year may be stored.

The process conditions such as humidity and acidity may be controlled in each cell separately. In such a plan cells are used successively, wherein the time interval between cell completion and gas production may be reduced considerably.

The humidity in the depot may be controlled in different ways. However, a control by means of adjustment of the water level in the depot is preferred.

Apart from controlling the humidity by varying the water level, the humidity may be controlled also by means of recycling leachate. In the above disclosed microbiological fermentations water is formed that preferably may be separated from the biogas formed and is drained away separately. However, it may be desired to recycle the leachate on one side to maintain the humidity at the level of at least 40 weight % and on the other side to control the pH-value. The pH-value effectively may be maintained below 8 by means of recycling leachate, since untreated leachate contains water soluble fatty acids that have been formed during the fermentation and consequently has a pH of less than 7.

By means of alkaline materials the pH of the depot may be maintained at a value of at least 6. Such alkaline material may be applied as a bottom cover in a cell, but they may be added also as such to the depot. However, it is also very effective to add alkaline materials to a part of the leachate, so that the pH-value of the leachate pass 7 and the leachate obtained is recycled.

When the fermentation under anaerobic conditions has been completed, the residue formed is dried by adjusting the water level in the depot as low as possible and by passing air through said residu and carrying off through a biogas collecting system. By means of sieving a substantially dry malodour-free compost is separated from the residue.
Preferably, the air to be carried off is filtrated through a heap of
compost. The substantial completion of the conversion appears from the
decrease of the biogas production and the low fatty acid contents of
the leaching water.

EXAMPLE

By means of a mechanical pretreatment the coarse and heavy fraction
of the municipal solid waste is separated from the light, predominantly
organic fraction.

The mechanical pretreatment consists of the following parts:

- block house with crane.
  Apart from the required buffering capacity, in this way a well
  ordered feed, with respect to the quantity, as well as with respect
  to the control of the material fed, is achieved.
- feeding system having restrictions with respect to the size and the
  weight.
  What has been escaped to the attention of the machinist, is kept out
  of the installation in this way.
- a sack tearing up device.
- since the ferro-components have to be removed from a stream that has
  not been subjected to a comminuting operation an upperband magnete is
  used.
- cutting machine.
  The cutting is not carried out very far.
- air separation.
  In said phase the "light" and the "heavy" parts are separated.
  Since the separation should not be that sharp a high air speed
  respectively low blowing out speed is sufficient.
  A cloth filter avoids that solid particles are carried off together
  with the air.
  The organic fraction is fed to a gas winning depot.

A cell-like depot is used, wherein each cell may store the feed of
organic material of at least one year.

The organic materials are tipped into the cell and subsequently
they are a bit densified. In order to control the acidity simultaneously alkaline materials are added in such an amount that in the starting
phase of the fermentation process the acidity remains neutral.

As soon as a cell has been filled up completely, a distribution system for wetting by means of leachate is applied. Then the cell is covered with a layer of soil, having a thickness of about 30 cm. Subsequently fed refuse materials are put in the next cell. In the completed cell the anaerobic fermentation is initiated. This is achieved in first instance by increasing the humidity. For that purpose an artificial water level is adjusted in the cell. The water required therefore is drawn partially from another cell and partially from outside. Then recycling of said leachate is started, wherein in the collecting pump-well still an adjustment of the acidity is performed. In this way the initial phase is reduced considerably, so that the gas production may take place quickly. The gas is exhausted by means of a vacuum via the collecting pump-well.

The gas-forming phase is a multi-years phase. The gas production is controlled by means of measurement of pressure difference under the covering soil layer. Dependent on the results of the measurement the vacuum as well as the water circulation are controlled. In this way a relatively constant production may be achieved.

In the course of time, a considerable part of the organic material will be fermented by means of the fermentation process, so that setting of the material put down takes place. It is for that reason that a flexible water distribution system and a partial perforation of the walls of the gas carrying off shafts are used.

After 7 years the gas production has been reduced considerably and exhaustion is stopped. The water present is pumped to another cell or into a sewer pipe. The soil layer is removed substantially completely. Subsequently air is passed through the fermented material. In this way a further drying of the material as well as an after fermentation of the organic residu is achieved.

The air exhausted is subjected to an after-treatment by means of a compost filter to avoid malodour.

When said phase has been completed, the dried and matured residu is subjected to a sieve treatment. The compost obtained in this way is processed further. The sieve residue is separated further in a pre-separating device into a plastic fraction, an organic fraction and an inert fraction.

The organic fraction is, if desired, after grinding, fed to another depot.

Subsequently the empty cell is used for a new cycle.

During a period of 7 years the biogas production takes place in the depot.

Totally about 150 $m^3$ of biogass is formed per ton of material fed.

The biogas has a sufficient high energy capacity to be used for heat production or for heat-energy coupling.

Dependent on the use the biogas should be subjected to a drying operation, compression operation and, if desired, a sulfide purification.

The fermented residue in the depot consists predominantly of a stable compost.

Since iron and non-ferro components have been separated therefrom already in the mechanical pre-separation, the compost is as far as it regards to heavy metals, of a good quality.

The crude compost is still polluted with 5 weight % (starting value) of plastics and about 5 weight % of non-compostable materials. Separation of said materials by means of sieving is very well possible. Finally about 20 weight % of compost is obtained.

## CLAIMS

1. A process for the anaerobic fermentation of the organic fraction of municipal solid waste, by subjecting said fraction to a treatment, wherein after a brief fermentation under aerobic conditions the oxygen present is consumed by aerobic microorganisms, carbohydrates, fats and proteins are hydrolyzed under anaerobic conditions by means of facultatively anaerobic microorganisms and subsequently the hydrolysis products formed, are fermented, likewise under anaerobic conditions, by means of strictly anaerobic microorganisms predominantly into methane and carbon dioxide, characterized in that in a depot of the organic fraction, that has been obtained after removal of non-fermentable components from municipal solid waste, the humidity is adjusted at at least 40 weight %, the pH-value is adjusted at 6 to 8, said conditions are maintained, and the residue formed in the depot is removed.

2. A process according to claim 1, characterized in that the depot is formed in a cell wherein the refuse feed of at least one year may be stored.

3. A process according to claim 1 or 2, characterized in that the humidity in the depot is controlled by means of adjusting the water level.

4. A process according to claim 1-3, characterized in controlling the humidity in the depot by means of recycling leachate.

5. A process according to claim 1-4, characterized in carrying off separately the biogas and leachate formed in the depot.

6. A process according to claim 1-5, characterized in maintaining the pH-value in the depot at at least 6 by adding alkaline materials.

7. A process according to claim 1-6, characterized in maintaining the pH below 8 by adding leachate to the depot.

8. A process according to claims 1-7, characterized in maintaining the temperature of the depot within the range of from 25 to 60°C.

9. A process according to claim 1-8, characterized in drying the residue by means of decreasing the water level in the depot and/or passing air through it, and separating the compost from said residue.

-.-.-.-.-

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | WATER, AIR AND SOIL POLLUTION, vol. 2, 1973, pages 483-495, D. Reidel Publishing Company, Dordrecht, NL. G.J. FARQUHAR et al.: "Gas production during refuse decomposition" * Page 483, paragraphs 3-5; page 484, paragraph 3; figure 1; page 488, paragraphs 4-6; page 489, paragraph 6; page 490, paragraphs 1,2; page 491, paragraphs 3-5; figure 3; page 493, paragraph 2; summary and conclusions * | 1-5,7, 8 | C 05 F 9/04 C 12 P 5/02 |
| Y | DE-A-2 930 418 (ING. WERNER WEBER INGENIEUR-GESELLSCHAFT) * Figures; claims 1,4-6 * | 1-5,7, 8 | |
| D,Y | GB-A-1 341 305 (S.F.R.I.R.) * Claim 1; page 2, line 77 - page 3, line 28; example * | 1,6,8 | TECHNICAL FIELDS SEARCHED (Int. Cl. 3) |
| A | FR-A-2 074 585 (D. ADAM) * Page 1, line 22 - page 4, line 16 * | 1,6,8 | C 05 F C 12 P B 09 B |
| A | FR-A- 902 439 (A.M. TANNIER) * Page 1, line 45 - page 2, line 62; figures * | 1,3-8 | |
| | --- -/- | | |

The present search report has been drawn up for all claims

| Place of search | Date of completion of the search | Examiner |
|---|---|---|
| THE HAGUE | 01-08-1983 | COUCKE A.O.M. |

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | DE-A-3 015 239 (KNEER FRANZ XAVER) * Claim 1 * | 1,3,8 | |

-----

TECHNICAL FIELDS SEARCHED (Int. Cl. 3)

The present search report has been drawn up for all claims

| Place of search | Date of completion of the search | Examiner |
|---|---|---|
| THE HAGUE | 01-08-1983 | COUCKE A.O.M. |